(19) Europäisches Patentamt
European Patent Office
Office européen des brevets

(11) Numéro de publication: **0 200 591**
**B1**

# (12) FASCICULE DE BREVET EUROPEEN

(45) Date de publication du fascicule du brevet: **18.01.89**

(51) Int. Cl.[4]: **C 07 J 11/00**, C 07 J 31/00

(21) Numéro de dépôt: **86400622.6**

(22) Date de dépôt: **25.03.86**

(54) **Nouveau procédé de préparation de dérivés de l'androstène-2 à l'état pur.**

(30) Priorité: **29.04.85 FR 8506471**

(43) Date de publication de la demande:
**05.11.86 Bulletin 86/45**

(45) Mention de la délivrance du brevet:
**18.01.89 Bulletin 89/3**

(84) Etats contractants désignés:
**AT BE CH DE FR GB IT LI LU NL SE**

(56) Documents cités:
**EP-A- 0 066 001**
**FR-A- 2 258 185**
**US-A- 3 098 851**

**JOURNAL OF THE CHEMICAL SOCIETY, Chemical Communications, vol. 3, 1969, page 112, Londres, GB; M. FETIZON et al.: "A new synthesis of delta 2-steroids"**

(73) Titulaire: **LABORATOIRE THERAMEX, 2 Boulevard Charles III, Monaco (MC)**

(72) Inventeur: **Grignard, Robert, 2 rue Arson, F-06300 Nice (FR)**

(74) Mandataire: **Burtin, Jean-François, 5 Bis, rue Parmentier, F-92200 Neuilly S/Seine (FR)**

EP 0 200 591 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

Un procédé d'obtention d'un composé Δ-2 androsténique à l'état pur à partir d'un mélange de Δ-2(3) et de Δ-3(4) androstène caractérisé en ce que l'on soumet un mélange d'androstènes répondant à la formule générale

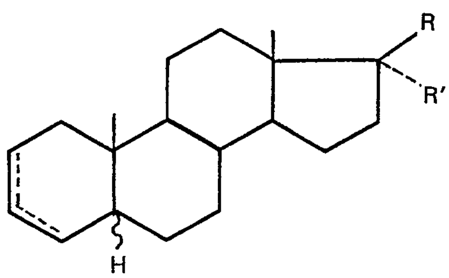

dans laquelle
le trait pointillé symbolise une double liaison en postion 2(3) ou en position 3(4)
R représente un hydroxyle libre, estérifié par un acide organique ayant de 1 à 10 atomes de carbone, ou éthérifié par un radical alcoyle ou tétrahydro-pyranyle
R' représente de l'hydrogène, un radical alcényle inférieur, un radical alcynyle inférieur, un radical alcoyle inférieur, un radical phényle ou un radical cyclo-alcoyle
ou bien R et R' forment, ensemble, l'oxygène d'une fonction cétone,
et le trait ondulé indique une orientation α ou β à l'action d'un agent de sulfonation pur ou dans un solvant inerte en présence éventuelle d'un agent de déshydratation pour obtenir un acide Δ-3 androstény1 2-sulfonique de formule générale II

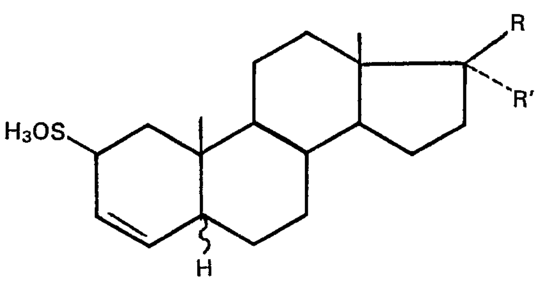

(II)

dans laquelle R et R' ont les significations fournies antérieurement
et récupère le dérivé Δ-2(3) androsténique pur, non transformé, de formule générale IV

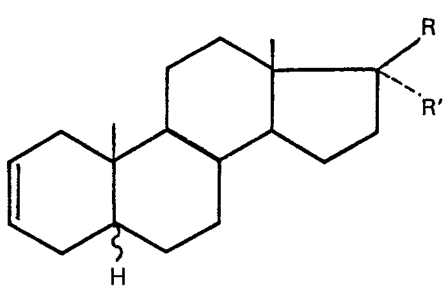

(IV)

dans laquelle les substituants R et R' sont définis comme précedemment.

La préparation de ces dérivés est déjà connue et s'effectue selon les procédés décrits, principalement, soit par élimination du tosylate en 3 du stéroïde correspondant (brevet des Etats-Unis d'Amérique 3 098 851), soit par pyrolyse d'un 3-spiro-3-diazirino stéroïde (brevet français n° 1 487 671), mais les dérivés obtenus par ces techniques renferment toujours une proportion importante de l'isomère Δ-3 androsténique. Il existe aussi un procédé de purification du 17-oxo-5α-androstène-2, décrit par la demanderesse (dans la demande de brevet français n° 74-43 397) qui consiste à oxyder l'isomère Δ-3 par réactif de Jones (anhydride chromique en solution hydrosulfurique), les produits de dégradation étant ensuite éliminés par chromatographie sur silice en milieu benzénique. Ce procédé bien que séduisant, présente néanmoins trois inconvénients majeurs, à savoir, son rendement trop faible, son temps d'exécution trop long, ne serait-ce que par la chromatographie et enfin, l'utilisation de grandes quantités de benzène, solvant particulièrement toxique.

La présente invention repose sur la constatation surprenante qu'il est possible de sulfoner sélectivement en milieu anhydre l'isomère Δ-3 androsténique afin d'obtenir un dérivé fortement acide de hydrosoluble, répondant la formule générale II

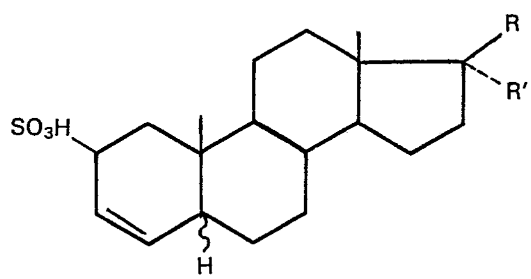

(II)

dans laquelle les substituants R et R' ont les significations fournies antérieurement.

Ce dérivé est ensuite facilement éliminé par des résines échangeuses d'anions en milieu alcoolique ou simplement en versant le milieu réactionnel dans de l'eau, opération qui provoque la cristallisation du stéroïde Δ-2 insoluble, non transformé, et la mise en solution du stéroïde Δ-3 sulfoné. Après filtration, lavages à l'eau et séchage, on obtient le stéroïde Δ-2 pur. La sulfonation par l'acide sulfurique, pur ou en solution, s'effectue dans un solvant inerte comme par exemple l'acide acétique, le dichlorométhane, le chloroforme, le dioxanne, en présence d'un agent déshydratant tel qu'un anhydride d'acide alcoylcarboxylique comme l'anhydride acétique ou l'anhydride propionique. La température du milieu réactionnel peut varier de −5°C à +50°C et le temps de réaction, de quelques minutes a plusieurs jours, suivant les conditions opératoires.

L'analyse structurale du stéroïde purifié a été effectuée par spectrophotométrie dans l'infrarouge et par résonance magnétique nucléaire du proton.

Les protons oléfiniques en C2 et C3 se présentent sous forme d'un singulet élargi à 334 Hz (largeur à mi-hauteur 5 Hz) alors que pour les dérivés Δ-3 androsténiques, les protons oléfiniques apparaissent sous forme de 4 massifs entre 312 et 348 Hz.

L'analyse quantitative de l'isomère Δ-3 androsténique a été effectuée par HPLC après oxydation par l'anhydride chromique ($CrO_3$) en cétone α-conjuguée qui est détectée à 240 nanomètres. Cette technique, sensible et précise, a permis de montrer, par les exemples qui vont suivre, que le taux de l'isomère Δ-3 dans les stéroïdes purifiés est inférieur à 1%.

Les contrôles de pureté ont été effectués par chromatographie sur couche mince dans le système solvant: toluène 75 ml - acétate d'éthyle 25 ml puis révélation par une solution éthanolique d'acide sulfurique à 20% V/V et chauffage 1 minute à 110°C.

L'invention concerne également les acides Δ-3-androstény1-2-sulfoniques de formule générale II, formés au cours de l'exécution du procédé selon l'invention.

*EXEMPLE 1*

*17-oxo-5α-androstène-2*

On dissous 10 g de 17-oxo-5α-androstène impur renfermant 90% de l'isomère Δ-2 et 10% de l'isomère Δ-3 dans 20 ml du mélange solvant: dichlorométhane 50 ml - anhydride acétique 50 ml. On maintient la température à 8°C à l'aide d'un bain de glace et on ajoute, sous agitation, en 30 minutes, 20 ml de réactif préparé en additionnant 0,6 ml d'acide sulfurique concentré à 20 ml du mélange solvant précédent. On verse rapidement le milieu réactionnel dans 1 litre d'eau et laisse agiter 4 heures à température ambiante. On filtre, lave abondamment à l'eau et sèche les cristaux 4 heures sous vide à 70°C.

On obtient 8 g de 17-oxo-5α-androstène-2.

| | |
|---|---|
| • rendement pondéral | 80% |
| • rendement théorique | 88% |
| • C.C.M. | monotache |
| • spectre I.R. | conforme à la structure |
| • spectre de R.M.N. | conforme à la structure isomère Δ-3 non visible |
| • dosage HPLC du Δ-3 | 0,4% |

*EXEMPLE 2*

*17-oxo-5α-androstène-2*

20 g de 17-oxo-5α-androstène impur renfermant 86% de l'isomère Δ-2 et 14% de l'isomère Δ-3 ont été dissous dans 40 ml du mélange solvant: dichlorométhane 30 ml - anhydride acétique 10 ml - acide acétique 60 ml. On maintient la température à 5°C à bain de glace et on ajoute, sous agitation, 40 ml de réactif sulfonat préparé en additionnant 2 ml d'acide sulfurique concentré à 38 ml du mélange solvant. La durée d'addition du réactif a été de 30 minutes et on laisse agiter encore 15 minutes avant de verser le milieu réactionnel dans 2 litres d'eau. On agite 2 heures, filtre, lave abondamment à l'eau et sèche 2 heures sous vide à 80°C.

On obtient 17,2 g de 17-oxo-5α-androstène-2.

| | |
|---|---|
| • rendement pondéral | 86% |
| • rendement théorique | 99% |
| • point de fusion | 101°C |
| • C.C.M. | monotache |
| • spectre I.R. | conforme à la structure |
| • spectre de R.M.N. | conforme à la structure isomère Δ-3 non visible |
| • dosage HPLC du Δ-3 | 0,8% |

*EXEMPLE 3*

*17-oxo-5α-androstène-2*

10 g de 17-oxo-5α-androstène impur renfermant 80% de l'isomère Δ-2 et 20% de l'isomère Δ-3 ont été dissous dans 50 ml du mélange solvant: dichlorométhane 75 ml - anhydride acétique 35 ml. La température à été maintenue à 5°C à l'aide d'un bain de glace - 50 ml de réactif, préparé en ajoutant 1,5 ml d'acide sulfurique à 48,5 ml du mélange solvant ont été ajoutés en 30 minutes.

Le milieu réactionnel a été versé rapidement dans 300 ml d'éthanol anhydre, et après 2 heures d'agitation, la solution obtenue a été passée sur une colonne chargée avec 50 g de résine échangeuse d'anions, DOWEX® AG2 X8 (sous forme $OH^-$) préalablement déshydratée à l'éthanol anhydre. Après passage de la solution, la colonne a été lavée avec 100 ml d'éthanol, les effluents et les liqueurs de lavage réunis ont été évaporée à sec sous vide.

On obtient 7,74 g de 17-oxo-5α-androstène-2.

| | |
|---|---|
| • rendement pondéral | 77,4% |
| • rendement théorique | 97% |
| • point de fusion | 101,4°C |
| • C.C.M. | une tache secondaire à faible intensité |
| • spectre I.R. | conforme à la structure |
| • spectre de R.M.N. | conforme à la structure isomère Δ-3 non visible |
| • dosage HPLC du Δ-3 | 0,7% |

*EXEMPLE 4*

*17-oxo-5α-androstène-2*

On dissous 10 g de 17-oxo-5α-androstène impur renfermant 83% de l'isomère Δ-2 et 17% de l'isomère Δ-3 dans 20 ml du mélange solvant: dichlorométhane 90 ml - anhydride acétique 10 ml. On maintient la température à 0°C au réfrigérateur et ajoute rapidement, sous agitation magnétique, 20 ml de réactif préparé en additionnant 0,5 ml d'acide sulfurique concentré à 20 ml du mélange solvant. On laisse agiter 48 heures à 0°C et verse le milieu réactionnel dans 200 ml de méthanol. Après 2 heures d'agitation, on a ajouté 20 g de résine échangeuse d'anions, DOWEX® AG2 X8 (sous forme $OH^-$) préalablement déshydratée au méthanol et 30 minutes à température ambiante. On filtre, lave la résine avec 50 ml de méthanol et évapore à sec le filtrat et les liquides de lavage réunis.

On obtient 7,74 g de 17-oxo-5α-androstène-2.

| | |
|---|---|
| • rendement pondéral | 77,4% |
| • rendement théorique | 93% |
| • point de fusion | 100,2°C |
| • C.C.M. | monotache |
| • spectre I.R. | conforme à la structure |

| | |
|---|---|
| • spectre de R.M.N. | conforme à la structure isomère Δ-3 non visible |
| • dosage HPLC du Δ-3 | 0,5% |

*EXEMPLE 5*

*17-oxo-5α-androstène-2*

10 g de 17-oxo-5α-androstène impur renfermant 86,5% de l'isomère Δ-2 et 13,5% de l'isomère Δ-3 ont été dissous dans 50 ml de dioxanne. On ajoute sous bonne agitation 10 ml d'anhydride acétique, 0,8 ml d'acide sulfurique concentré et laisse agiter 15 minutes à température ambiante (22°C environ). On verse le milieu réactionnel dans 1 litre d'eau et laisse agiter 16 heures. On filtre, lave abondamment à l'eau et sèche les cristaux 4 heures sous vide à 80°C.

On obtient 8,1 g de 17-oxo-5α-androstène-2.

| | |
|---|---|
| • rendement pondéral | 81% |
| • rendement théorique | 93,6% |
| • point de fusion | 101,8°C |
| • C.C.M. | monotache |
| • spectre I.R. | conforme à la structure |
| • spectre de R.M.N. | conforme à la structure isomère Δ-3 non visible |
| • dosage HPLC du Δ-3 | 0,1% |

*EXEMPLE 6*

*17-oxo-5α-androstène-2*

On dissout 5 g de 17-oxo-5α-androstène impur renfermant 86,5% de l'isomère Δ-2 et 13,5% de l'isomère Δ-3 dans 10 ml de chloroforme. On ajoute sous bonne agitation 2 ml d'anhydride acétique puis 0,8 ml d'acide sulfurique concentré et maintient l'agitation 15 minutes à température ambiante. On verse le milieu réactionnel dans 100 ml de méthanol et, après 2 heures d'agitation, on ajoute 10 g de résine échangeuse d'anions. DOWEX® AG2 X8 (sous forme $OH^-$) préalablement déshydratée avec du méthanol, agite 30 minutes, filtre, lave avec 20 ml de méthanol et évapore à sec sous vide filtrat et liqueurs de lavage réunis.

On obtient 4 g de 17-oxo-5α-androstène-2.

| | |
|---|---|
| • rendement pondéral | 80% |
| • rendement théorique | 92,5% |
| • point de fusion | 102°C |
| • C.C.M. | monotache |
| • spectre I.R. | conforme à la structure |
| • spectre de R.M.N. | conforme à la structure isomère Δ-3 non visible |
| • dosage HPLC du Δ-3 | 0% |

*EXEMPLE 7*

*17-oxo-5α-androstène-2*

10 g de 17-oxo-5α-androstène impur renfermant 86,5% de l'isomère Δ-2 et 13,5% de l'isomère Δ-3 ont été dissous dans 50 ml de dioxanne. On ajoute sous bonne agitation 10 ml d'anhydride propionique, 0,8 ml d'acide sulfurique concentré et laisse agiter 15 minutes à température ambiante. On verse le milieu réactionnel dans 1 litre d'eau et laisse agiter 16 heures. On filtre, lave abondamment à l'eau et sèche les cristaux 6 heures sous vide à 65°C.

On obtient 8,15 g de 17-oxo-5α-androstène-2.

| | |
|---|---|
| • rendement pondéral | 81,5% |
| • rendement théorique | 94,2% |
| • point de fusion | 101°C |
| • C.C.M. | monotache |
| • spectre I.R. | conforme à la structure |
| • spectre de R.M.N. | conforme à la structure |
| • dosage HPLC du Δ-3 | 0,1% |

*EXEMPLE 8*

*17β-acétoxy-17α-éthinyl-5α-androstène-2*

10 g de 17β-acétoxy-17α-éthinyl-5α-androstène impur renfermant 83% de l'isomère Δ-2 et 17% de l'isomère Δ-3 ont été dissous dans 50 ml du mélange solvant: dichlorométhane 75 ml - anhydride acétique 25 ml. On ajoute, sous agitation, à température ambiante, en 30 minutes, 50 ml de réactif préparé par addition de 2,8 ml d'acide sulfurique concentré à 50 ml du mélange solvant. On verse le milieu réactionnel dans 2 litres d'eau et agite 16 heures à température ambiante. On filtre, lave abondamment à l'eau et sèche les cristaux 4 heures sous vide à 80°C.

On obtient 7,12 g de 17β-acétoxy-17α-éthinyl-5α-androstène-2.

| | |
|---|---|
| • rendement pondéral | 71,2% |
| • rendement théorique | 85,7% |
| • point de fusion | 126°C |
| • C.C.M. | monotache |
| • spectre I.R. | conforme à la structure |
| • spectre de R.M.N. | conforme à la structure isomère Δ-3 non visible |
| • dosage HPLC du Δ-3 | 0,9% |

*EXEMPLE 9*

*17β-acétoxy-17α-éthinyl-5α-androstène-2*

On dissout 10 g de 17β-acétoxy-17α-éthinyl-5α-androstène impur renfermant 86% de l'isomère Δ-2 et 14% de l'isomère Δ-3 dans 100 ml du mélange réactif: dichlorométhane 88,5 ml - anhydride acétique 10 ml - acide sulfurique concentré 1,5 ml. On agite 30 minutes à température ambiante et verse le milieu réactionnel dans 400 ml de méthanol. Après 2 heures d'agitation, on ajoute 20 g de résine échangeuse d'anions, DOWEX® AG2 XI (sous forme $OH^-$) préalablement déshydratée au méthanol et agite 2 heures. On filtre, lave la résine avec 200 ml de méthanol et évapore à sec sous vide filtrat et lavages réunis.

On obtient 8,5 g de 17β-acétoxy-17α-éthinyl-5α-androstène-2.

| | |
|---|---|
| • rendement pondéral | 85% |
| • rendement théorique | 97,7% |
| • point de fusion | 126,5°C |
| • C.C.M. | 1 tache secondaire à faible intensité |
| • spectre I.R. | conforme à la structure |
| • spectre de R.M.N. | conforme à la structure isomère Δ-3 non visible |
| • dosage HPLC du Δ-3 | 0,7% |

*EXEMPLE 10*

*17β-acétoxy-17α-éthinyl-5α-androstène-2*

10 g de 17β-acétoxy-17α-éthinyl-5α-androstène

impur renfermant 86% de l'isomère Δ-2 et 14% de l'isomère Δ-3 ont été dissous dans 20 ml de dichlorométhane. On ajoute rapidement, sous bonne agitation, 2 ml d'anhydride acétique, 1 ml d'acide sulfurique concentré, maintient l'agitation 2 minutes à température ambiante et verse le milieu réactionnel dans 1,5 litres d'eau. On agite 2 heures, filtre, lave abondamment à l'eau et sèche les cristaux 6 heures à 70°C.

On obtient 8,2 g de 17β-acétoxy-17α-éthinyl-5α-androstène-2.

- rendement pondéral 82%
- rendement théorique 95%
- point de fusion 126,4°C
- C.C.M. monotache
- spectre I.R. conforme à la structure
- spectre de R.M.N. conforme à la structure isomère Δ-3 non visible
- dosage HPLC du Δ-3 0,3%

*EXEMPLE 11*

*17β-propanoyloxy-17α-éthinyl-5α-androstène-2*

5 g de 17β-propanoyloxy-17α-éthinyl-5α-androstène impur renfermant 83% de l'isomère Δ-2 et 17% de l'isomère Δ-3 ont été dissous dans 10 ml de dichlorométhane. On ajoute, sous bonne agitation, 1 ml d'anhydride acétique, 0,8 ml d'acide sulfurique concentré, agite 5 minutes et verse le milieu réactionnel dans 1 litre d'eau. On agite 4 heures, filtre, lave abondamment à l'eau et sèche 4 heures sous vide à 70°C.

On obtient 3,9 g de 17β-propanoyloxy-17α-éthinyl-5α-androstène-2.

- rendement pondéral 78%
- rendement théorique 94%
- point de fusion 110°C
- C.C.M. monotache
- spectre I.R. conforme à la structure
- spectre de R.M.N. conforme à la structure isomère Δ-3 non décelable
- dosage HPLC du Δ-3 0,1%

*EXEMPLE 12*

*17β-butanoyloxy-17α-éthinyl-5α-androstène-2*

5 g de 17β-butanoyloxy-17α-éthinyl-5α-androstène impur renfermant 83% de l'isomère Δ-2 et 17% de l'isomère Δ-3 ont été dissous dans 10 ml de dichlorométhane. On ajoute, sous bonne agitation, 1 ml d'anhydride acétique, 0,8 ml d'acide sulfurique concentré, agite 5 minutes à température ambiante et verse le milieu réactionnel dans 1 litre d'eau. On agite 4 heures, filtre, lave abondamment à l'eau et sèche les cristaux 4 heures sous vide à 60°C.

On obtient 3,7 g de 17β-butanoyloxy-17α-éthinyl-5α-androstène-2.

- rendement pondéral 74%
- rendement théorique 89%
- point de fusion 80°C
- C.C.M. monotache
- spectre I.R. conforme à la structure
- spectre de R.M.N. conforme à la structure isomère Δ-3 non décelable
- dosage HPLC du Δ-3 0,2%

*EXEMPLE 13*

*17β-pentanoyloxy-17α-éthinyl-5α-androstène-2*

5 g de 17β-pentanoyloxy-17α-éthinyl-5α-androstène impur renfermant 83% de l'isomère Δ-2 et 17% de l'isomère Δ-3 ont été dissous dans 10 ml de dichlorométhane. On ajoute, sous bonne agitation, 1 ml d'anhydride acétique, 0,7 ml d'acide sulfurique concentré, agite 5 minutes à température ambiante et verse le milieu réactionnel dans 1 litre d'eau. On agite 4 heures, filtre, lave abondamment à l'eau et sèche 16 heures sous vide à 40°C.

On obtient 3,7 g de 17β-pentanoyloxy-17α-éthinyl-5α-androstène-2.

- rendement pondéral 74%
- rendement théorique 89%
- point de fusion 50°C
- C.C.M. 1 tache secondaire à faible intensité
- spectre I.R. conforme à la structure
- spectre de R.M.N. conforme à la structure isomère Δ-3 non décelable
- dosage HPLC du Δ-3 0,2%

*EXEMPLE 14*

*17β-heptanoyloxy-17α-éthinyl-5α-androstène-2*

5 g de 17β-heptanoyloxy-17α-éthinyl-5α-androstène impur renfermant 83% de l'isomère Δ-2 et 17% de l'isomère Δ-3 ont été dissous dans 10 ml de dichlorométhane. On ajoute, sous bonne agitation, 1 ml d'anhydride acétique, 0,7 ml d'acide sulfurique concentré, agite 5 minutes et verse le milieu réactionnel dans 100 ml de méthanol. Après 2 heures d'agitation, on ajoute 15 g de résine échangeuse d'anions DOWEX® AG2 X8 (sous forme $OH^-$), agite 1 heure, filtre, lave la résine avec 20 ml de méthanol et évapore à sec filtrat et lavage réunis.

On obtient 3,5 g de 17β-heptanoyloxy-17α-éthinyl-5α-androstène-2 se présentant sous forme d'huile jaune-clair.

- rendement pondéral 72%
- rendement théorique 86%
- point de fusion liquide à température ambiante
- C.C.M. 1 tache secondaire à faible intensité
- spectre I.R. conforme à la structure
- spectre de R.M.N. conforme à la structure isomère Δ-3 non décelable
- dosage HPLC du Δ-3 0,6%

*EXEMPLE 15*

*17β-hydroxy-17α-éthinyl-5α-androstène-2*

10 g de 17β-hydroxy-17α-éthinyl-5α-androstène impur renfermant 83% de l'isomère Δ-2 et 17% de l'isomère Δ-3 ont été dissous dans 20 ml de dichlorométhane. On ajoute, sous bonne agitation, 1 ml d'anhydride acétique, 1 ml d'acide sulfurique concentré et laisse 15 minutes sous agitation à température ambiante. Le milieu réactionnel a été versé dans 2 litres d'eau et après 4 heures d'agitation, on filtre, lave abondamment à l'eau et sèche les cristaux 2 heures sous vide à 90°C.

On obtient 7,65 g de 17β-hydroxy-17α-éthinyl-5α-androstène-2.

| | |
|---|---|
| • rendement pondéral | 76,5% |
| • rendement théorique | 92% |
| • point de fusion | 165°C |
| • C.C.M. | monotache |
| • spectre I.R. | conforme à la structure |
| • spectre de R.M.N. | conforme à la structure isomère Δ-3 non décelable |
| • dosage HPLC du Δ-3 | 0,3% |

La présente invention n'est pas limitée aux exemples de réalisation qui viennent d'être décrits, elle est, au contraire, susceptible de variantes et de modifications qui apparaîtront à l'homme de l'art.

Ainsi qu'il a été indiqué dans la littérature et notamment dans le brevet des Etats-Unis d'Amérique 4 278 668 (Gueritée), les dérivés Δ-2 androsténiques de formule générale IV sont des médicaments qui se comportent, selon les conditions d'utilisation, comme des freinateurs ou au contraire des stimulants hypophysaires.

Ils trouvent diverses applications thérapeutiques comme l'endométriose, la stérilité, certaines formes de réparation osseuse comme l'ostéoporose dans les maladies bénignes du sein et dans le domaine vétérinaire dans la pseudogestation de la chienne.

## Revendications

1. Un procédé d'obtention d'un composé Δ-2 androsténique à l'état pur à partir d'un mélange de Δ-2(3) et de Δ-3(4) androstène caractérisé en ce que l'on soumet un mélange d'androstènes répondant à la formule générale

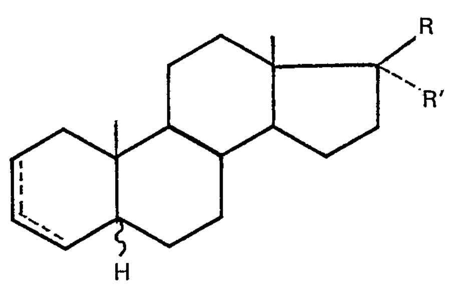

dans laquelle
le trait pointillé symbolise une double liaison en position 2(3) ou en position 3(4)
R représente un hydroxyle libre, estérifié par un acide organique ayant de 1 à 10 atomes de carbone, ou éthérifié par un radical alcoyle ou tétrahydropyranyle
R' représente de l'hydrogène, un radical alcényle inférieur, un radical alcynyle inférieur, un radical alcoyle inférieur, un radical phényle ou un radical cyclo-alcoyle
ou bien R et R' forment, ensemble, l'oxygène d'une fonction cétone,
et le trait ondulé indique une orientation α ou β
à l'action d'un agent de sulfonation pur ou dans un solvant inerte en présence éventuelle d'un agent de déshydratation pour obtenir un acide Δ-3 androstényl 2-sulfonique de formule générale II

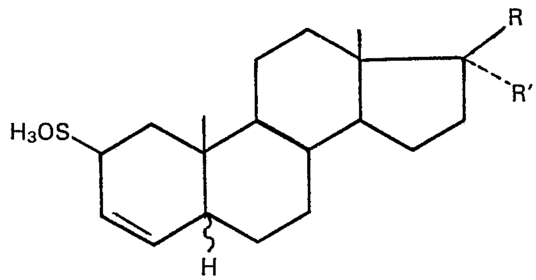

(II)

dans laquelle R et R' ont les significations fournies antérieurement
et récupère le dérivé Δ-2(3) androsténique pur, non transformé, de formule générale IV

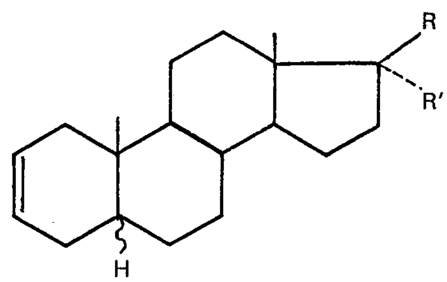

(IV)

dans laquelle les substituants R et R' sont définis comme précedemment.

2. Un procédé de préparation selon la revendication 1, dans lequel l'agent de sulfonation est l'acide sulfurique.

3. Un procédé de préparation selon la revendication 2, dans lequel le solvant inerte de la réaction de sulfonation est choisi parmi un acide aliphatique, un carbure halogéné, un éther cyclique et un éther linéaire.

4. Un procédé de préparation selon la revendication 2, dans lequel la sulfonation est effectuée en présence d'un agent déshydratant.

5. Un procédé de préparation selon la revendication 1, dans lequel la séparation de l'acide Δ-3 androstenyl 2-sulfonique est effectuée par adjonction d'un milieu aqueux qui le dissout.

6. Un procédé de préparation selon la revendication 1, dans lequel l'acide Δ-3 androstenyl 2-sulfonique est isolé par passage sur une résine échangeuse d'anions.

7. Un procédé de préparation selon la revendication 2, dans lequel l'agent déshydratant est un anhydride d'acide alcoyle carboxylique comme l'anhydride acétique ou l'anhydride propionique.

8. Les acides Δ-3 androsténypl-2-sulfoniques de formule générale II

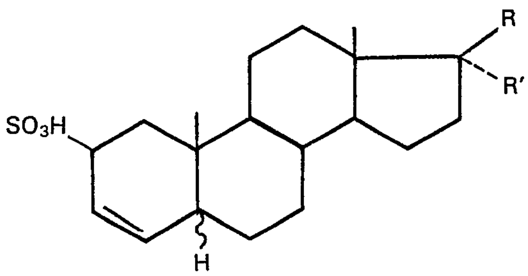

(II)

dans laquelle les substituants R et R' ont les définitions fournies ci-dessus
et le trait ondulé indique une configuration α ou β.

**Patentansprüche**

1. Verfahren für die Herstellung einer Δ-2 Androstenischen Verbindung im reinen Zustand von einem Gemisch von Δ-2(3) und Δ-3(4) Androstenen dadurch gekennzeichnet dass ein Gemisch von Androsten der allgemeinen Formel

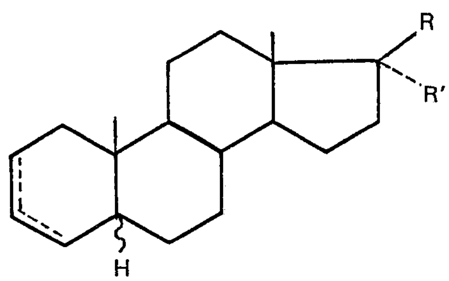

(I)

in der
die punktierte Linie eine Doppel Bindung in Stellung 2(3) oder 3(4) bedeutet
R für eine freie, mit einer organische $C_1$-$C_{10}$ Säure esterifierte oder mit einem Alkyl Radikal oder einem Tetrahydropyranyl etherifierte Hydroxyl steht
R' Wasserstoff, ein niedrig Alkenyl Radikal, ein niedrig Alkynyl Radikal, ein niedrig Alkyl Radikal, ein Phenyl Radikal ist
oder R und R' zusammen das Sauerstoff einer ketonische Funktion bilden
und die wellenförmige Linie eine α oder β räumliche Orientierung bedeutet
auf die Wirkung eines reinen oder in einem inerten Lösungsmittel Sulfonierungsmittel gegebenenfalls in Anwesenheit eines Dehydratisierungsmittel unterworfen wird, um eine Δ-3 Androstenyl 2-sulfonsäure der allgemeinen Formel II

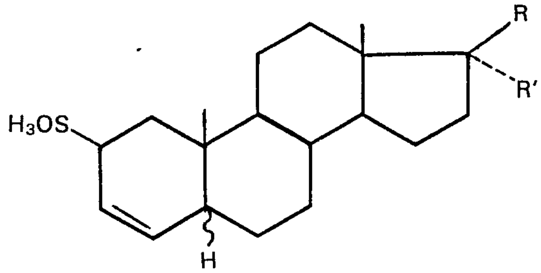

(II)

zu erlangen und die reine, unverwandelte Δ-2(3) androstenische Verbindung der allgemeinen Formel IV

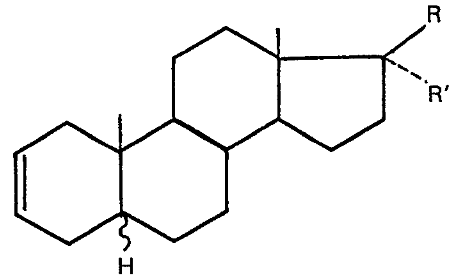

(IV)

worin die substituenten R und R' die selbe Bedeutungen wie vorher, besitzen zuruckgewonnen wird.

2. Verfahren zur Herstellung nach Anspruch 1, worin der Sulfonierungsmittel, Schwefelsäure ist.

3. Verfahren zur Herstellung nach Anspruch 2, worin das inertes Lösungsmittel für die Sulfonierungsumsetzung aus der Gruppe von einer aliphatischen Säure, eines Halogen-haltigen kohlenwasserstoff eines cyclischen Äther und eines linearen Äther gewählt ist.

4. Verfahren zur Herstellung nach Anspruch 2, worin die Sulfonierung in Anwesenheit eines Dehydrierungsmittel durchgeführt wird.

5. Verfahren zur Herstellung nach Anspruch 2, worin die Abtrennung der Δ-3 Androstenyl-2-sulfonsäure durch Zugabe eines wässerigen Mediums das sie auflöst, durchgeführt ist.

6. Verfahren zur Herstellung nach Anspruch 2, worin die Δ-3 Androstenyl-2-sulfonsäure durch auf einem Anionvertauscher Harz Durchgang, isoliert wird.

7. Verfahren zur Herstellung nach Anspruch 2, worin das Dehydrierungsmittel, das Anhydrid einer Alkyl carbonsäure wie Essigsäure Anhydrid oder Propionsäure Anhydrid, ist.

8. Die Δ-3 Androstenyl-2-sulfonsäure der allgemeinen Formel II

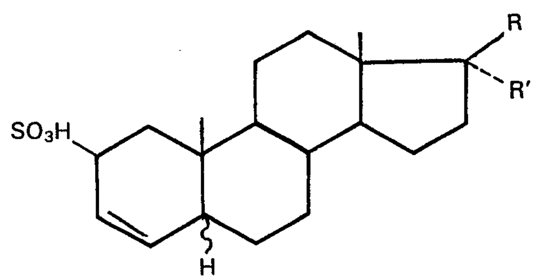

(II)

worin die substituenten R und R' die vorherige angegebene Bedeutungen haben, und die wellenförmige Linie eine α oder β räumliche Orientierung bedeutet.

**Claims**

1. A process for producing a Δ-2 androstenic derivative in the pure state form a mixture of Δ-2(3) and Δ-3(4) androstene characterized in that a mixture of Androstenes of the formula

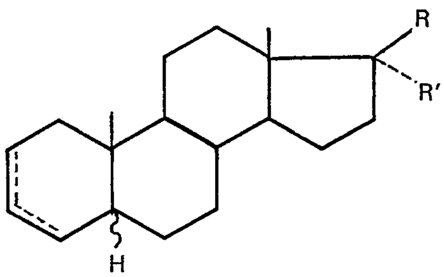

(I)

wherein
the dotted line symbolizes a double bond in the position 2(3) or 3(4)
R is a, free esterified with an organic acid having from 1 to 10 carbon atoms or etherified with an alkyl radical or a tetrahydropyranyl, hydroxyl group
R' is hydrogen, a lower alkenyl radical, a lower alkynyl radical, a lower alkyl radical a phenyl radical or a cycloalkyl radical or R and R' together are the oxygen of a ketonic group
and the wawed line indicates the spatial orientation α or β
is submitted to the action of a sulfonating agent pure or in an inert solvent in the optional presence of a dehydrating agent, to obtain a Δ-3 Androstenyl 2-sulfonic acid of the general formula II

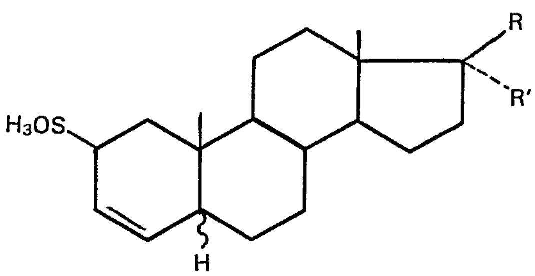

(II)

in which R and R' have the previously given meanings and recovers the pure Δ-2 androstenic derivative, unaltered having the general formula IV

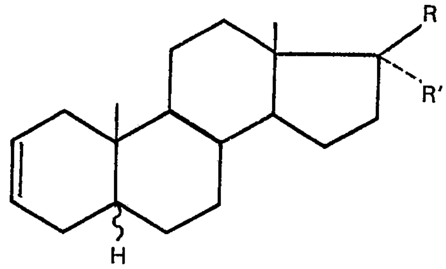

(IV)

wherein the
substituents R and R' are defined as previous by given.

2. A process of production according to claim 1 in which the sulfonating agent is sulphuric acid.

3. A process of production according to claim 2 in which the inert solvent for the sulfonation is selected from the group consisting of an aliphatic acid, a halogenated hydrocarbon, a cyclic ether and a linear ether.

4. A process of production according to claim 2 wherein the sulfonation is performed in the presence of a dehydrating agent.

5. A process of production according to claim 1 in which the isolation of Δ-3 androstenyl 2-sulphonic acid is performed by adding an aqueous medium which dissolves it.

6. A process of production according to claim 1 in which the Δ-3 androstenyl 2-sulphonic acid is isolated through passing on an an ion-exchange resin.

7. A process of production according to claim 2 in which the dehydrating agent is an alkylcarboxylic acid anhydride such as acetic or propionic anhydride.

8. The Δ-3 androstenyl sulphonic acids of general formula II

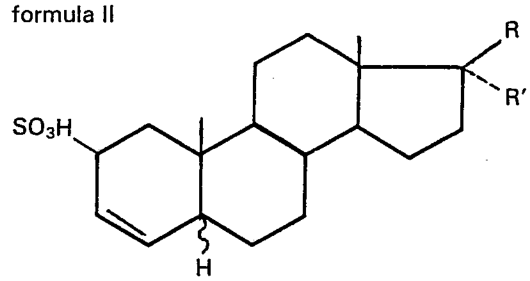

(II)

in which the substituents R and R' have the above-given definitions
and the wawed line indicates a spatial configuration α or β.